(19) Europäisches Patentamt
European Patent Office
Office européen des brevets

(11) Veröffentlichungsnummer: **0 414 076 B1**

# (12) EUROPÄISCHE PATENTSCHRIFT

(45) Veröffentlichungstag der Patentschrift: **12.10.94**

(51) Int. Cl.$^5$: **C07D 215/14**, C07D 409/12, A61K 31/47

(21) Anmeldenummer: **90115442.7**

(22) Anmeldetag: **11.08.90**

Die Akte enthält technische Angaben, die nach dem Eingang der Anmeldung eingereicht wurden und die nicht in dieser Patentschrift enthalten sind.

## (54) Cyclisch substituierte (Chinolin-2-yl-methoxy)phenyl-essigsäure-Derivate.

(30) Priorität: **24.08.89 DE 3927930**

(43) Veröffentlichungstag der Anmeldung:
**27.02.91 Patentblatt 91/09**

(45) Bekanntmachung des Hinweises auf die Patenterteilung:
**12.10.94 Patentblatt 94/41**

(84) Benannte Vertragsstaaten:
**AT BE CH DE DK ES FR GB GR IT LI LU NL SE**

(56) Entgegenhaltungen:
**EP-A- 0 181 568**
**EP-A- 0 200 101**
**EP-A- 0 318 093**
**EP-A- 0 344 519**
**US-A- 4 769 461**

(73) Patentinhaber: **BAYER AG**

**D-51368 Leverkusen (DE)**

(72) Erfinder: **Mohrs, Klaus, Dr.**
**Claudiusweg 9**
**D-5600 Wuppertal 1 (DE)**
Erfinder: **Raddatz, Siegfried, Dr.**
**Jakob-Böhme-Strasse 21**
**D-5000 Köln 80 (DE)**
Erfinder: **Fruchtmann, Romanis,**
**Konrad-Adenauer-Ufer 79**
**D-5000 Köln 1 (DE)**
Erfinder: **Kohlsdorfer, Christian, Dr.**
**Franz-Stryck-Strasse 16**
**D-5042 Erftstadt (DE)**
Erfinder: **Müller-Peddinghaus, Reiner, Prof.**
**Klutstein 22a**
**D-5060 Bergisch-Gladbach 2 (DE)**
Erfinder: **Theisen-Popp, Pia, Dr.**
**Hermann-Löns-Strasse 64-66**
**D-5060 Bergisch-Gladbach 2 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft cyclisch substituierte (Chinolin-2-yl-methoxy)phenyl-essigsäurederivate, Verfahren zu ihrer Herstellung und ihre Verwendung in Arzneimitteln.

In EP-A2 181 568 werden meta-substituierte 3-(Chinolin-2-yl-methoxy)phenylessigsäuren und 2-[3-(Chinolin-2-yl-methoxy)phenyl]propionsäuren und deren Ester beschrieben. Ferner ist bekannt, daß substituierte (Chinolin-2-yl-methoxy)phenylderivate eine antiinflammatorische und antiallergische Wirkung besitzen [vgl. EP-A3 110 405].

EP-A3-0 200 101 beschreibt Verbindungen, die eine Chinolylmethoxyphenyl-Gruppe enthalten und eine lipoxygenaseinhibierende Wirkung aufweisen.

Es wurden nun neue cyclisch substituierte (Chinolin-2-yl-methoxy)phenylessigsäure-Derivate der allgemeinen Formel (I),

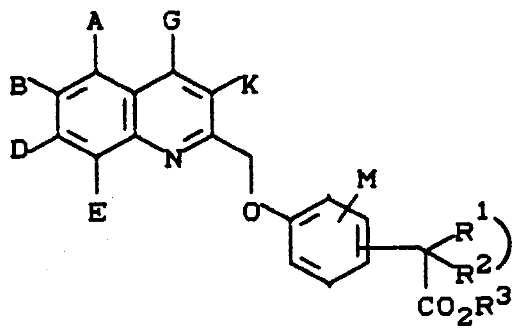

(I)

in welcher

A, B, D, E, G, K und M gleich oder verschieden sind und
- für Wasserstoff, Hydroxy, Halogen, Trifluormethyl, Trifluormethoxy oder Carboxy stehen,
- für geradkettiges oder verzweigtes Alkyl mit bis zu 10 Kohlenstoffatomen stehen, das gegebenenfalls durch Hydroxy oder Halogen substituiert ist,
- für geradkettiges oder verzweigtes Alkoxy oder Alkoxycarbonyl mit bis zu 10 Kohlenstoffatomen stehen, oder
- für Aryl mit 6 bis 10 Kohlenstoffatomen stehen, das gegebenenfalls durch Halogen, Nitro, Cyano oder durch geradkettiges oder verzweigtes Alkyl oder Alkoxy mit bis zu 8 Kohlenstoffatomen substituiert ist,

$R^1$ und $R^2$ gemeinsam einen 4- bis 8-gliedrigen, gesättigtigten oder ungesättigten Carbocyclus bilden, der gegebenenfalls bis zu 3-fach gleich oder verschieden durch geradkettiges oder verzweigtes Alkyl oder Alkoxy mit jeweils bis zu 8 Kohlenstoffatomen, Halogen, Hydroxy oder Aryl mit 6 bis 10 Kohlenstoffatomen substituiert ist, oder

$R^1$ und $R^2$ gemeinsam mit dem Kohlenstoffatom für einen Rest der Formel

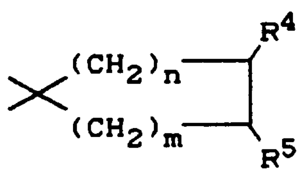

stehen,

worin

m und n gleich oder verschieden sind und eine Zahl 0, 1, 2, 3 oder 4 bedeuten,

und

$R^4$ und $R^5$ gemeinsam Aryl mit 6 bis 10 Kohlenstoffatomen oder einen 5- bis 7-gliedrigen, gesättigten oder ungesättigten Heterocyclus mit bis zu 3 Heteroatomen aus der Reihe Stickstoff, Schwefel oder Sauerstoff bilden, die gegebenenfalls durch geradkettiges oder verzweigtes Alkyl oder Alkoxy mit jeweils bis zu 8 Kohlenstoffatomen, Halogen oder Aryl mit 6 bis 10 Kohlenstoffatomen substituiert sind,

$R^3$ - für Wasserstoff, geradkettiges oder verzweigtes Alkyl mit bis zu 8 Kohlenstoffatomen oder Phenyl steht

gefunden und deren Salze.

Im Rahmen der vorliegenden Erfindung werden physiologisch unbedenkliche Salze bevorzugt. Physiologisch unbedenkliche Salze der cyclisch substituierten (Chinolin-2-yl-methoxy)phenyl-essigsäure-Derivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Bevorzugt sind Verbindungen der allgemeinen Formel (I),

in welcher

A, B, D, E, G, K und M gleich oder verschieden sind und

- für Wasserstoff, Fluor, Chlor, Trifluormethoxy oder Carboxy stehen,
- für geradkettiges oder verzweigtes Alkyl mit bis zu 8 Kohlenstoffatomen stehen, das gegebenenfalls durch Hydroxy, Fluor, Chlor oder Brom substituiert ist,
- für geradkettiges oder verzweigtes Alkoxy oder Alkoxycarbonyl mit bis zu 8 Kohlenstoffatomen stehen, oder
- für Phenyl stehen, das gegebenenfalls durch Fluor, Chlor, Brom, Nitro, Cyano oder durch geradkettiges oder verzweigtes Alkyl oder Alkoxy mit bis zu 6 Kohlenstoffatomen substituiert ist,

$R^1$ und $R^2$ gemeinsam für Cyclopropyl, Cyclobutyl, Cyclopentyl, Cyclohexyl, Cycloheptyl, Cyclopentenyl, Cyclohexenyl oder Cycloheptenyl stehen, die gegebenenfalls bis zu 2-fach gleich oder verschieden durch geradkettiges oder verzweigtes Alkyl mit bis zu 6 Kohlenstoffatomen oder Phenyl substituiert sind, oder

$R^1$ und $R^2$ gemeinsam mit dem Kohlenstoffatom für einen Rest der Formel

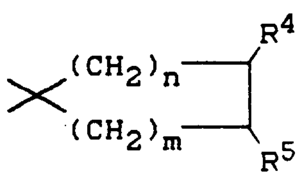

stehen,

worin

m und n gleich oder verschieden sind und eine Zahl 0, 1, 2 oder 3 bedeuten,

$R^4$ und $R^5$ gemeinsam Phenyl, Furanyl, Thienyl, Pyridyl oder Pyrryl bedeuten, die gegebenenfalls durch geradkettiges oder verzweigtes Alkyl oder Alkoxy mit jeweils bis zu 6 Kohlenstoffatomen oder Fluor, Chlor, Brom oder Phenyl substituiert sind,

$R^3$ - für Wasserstoff, geradkettiges oder verzweigtes Alkyl mit bis zu 8 Kohlenstoffatomen oder Phenyl steht

und deren Salze.

Besonders bevorzugt sind Verbindungen der allgemeinen Formel (I),

in welcher

A, B, D, E, G, K und M gleich oder verschieden sind und

- für Wasserstoff, Fluor, Chlor, geradkettiges oder verzweigtes Alkyl oder Alkoxy mit bis zu 6 Kohlenstoffatomen stehen,

$R^1$ und $R^2$ gemeinsam für Cyclopentyl, Cyclopentenyl, Cyclohexyl, Cyclohexenyl, Cycloheptyl oder Cycloheptenyl stehen, die gegebenenfalls durch geradkettiges oder verzweigtes Alkyl mit bis zu 4 Kohlenstoffatomen und/oder Phenyl

substituiert sind, oder
$R^1$ und $R^2$ gemeinsam mit dem Kohlenstoffatom für einen Rest der Formel

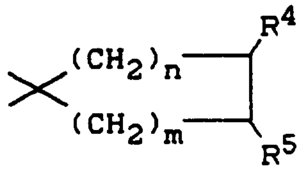

stehen,
worin

m und n gleich oder verschieden sind und eine Zahl 1 oder 2 bedeuten,

$R^4$ und $R^5$ gemeinsam Phenyl, Thienyl, Pyridyl oder Pyrryl bedeuten, die gegebenenfalls durch geradkettiges oder verzweigtes Alkyl mit bis zu 4 Kohlenstoffatomen oder Phenyl substituiert sind,

$R^3$ - für Wasserstoff oder geradkettiges oder verzweigtes Alkyl mit bis zu 6 Kohlenstoffatomen steht

und deren Salze.

Ganz besonders bevorzugt sind Verbindungen der allgemeinen Formel (I), in denen die Chinolylmethoxygruppierung am Phenyl in 4-Stellung zum cyclisch-substituierten Essigsäurerest steht.

Außerdem wurde ein Verfahren zur Herstellung der erfindungsgemäßen Verbindungen der allgemeinen Formel (I)

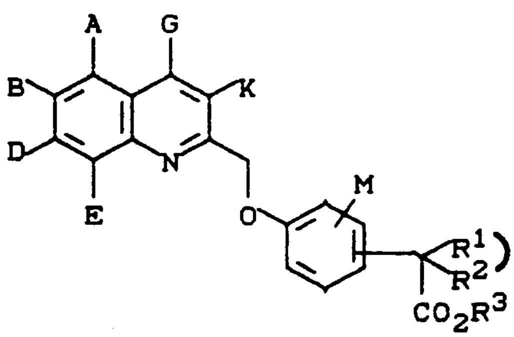

(I)

in welcher
A, B, D, E, G, K, M, $R^1$, $R^2$ und $R^3$ die oben angegebene Bedeutung haben
gefunden, das dadurch gekennzeichnet ist, daß man CH-acide-Verbindungen der allgemeinen Formel (II)

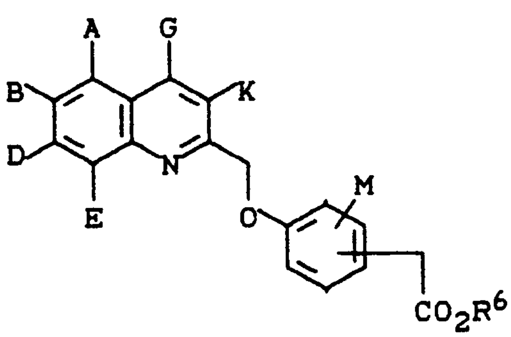

(II)

in welcher
A, B, D, E, G, K und M die oben angegebene Bedeutung haben,
und

$R^6$ - die oben angegebene Bedeutung von $R^3$ hat aber nicht für Wasserstoff steht,

mit Dihalogen-Verbindungen der allgemeinen Formel (III)

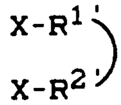

(III)

in welcher

$R^{1'}$ und $R^{2'}$ die oben angegebene Bedeutung von $R^1$ und $R^2$ haben, aber in der offenkettigen Form vorliegen,

und

X - für Halogen steht,

in inerten Lösemitteln, gegebenenfalls in Anwesenheit einer Base umsetzt, und dann im Fall der Säuren die Ester verseift.

Das Verfahren kann durch folgendes Formelschema erläutert werden:

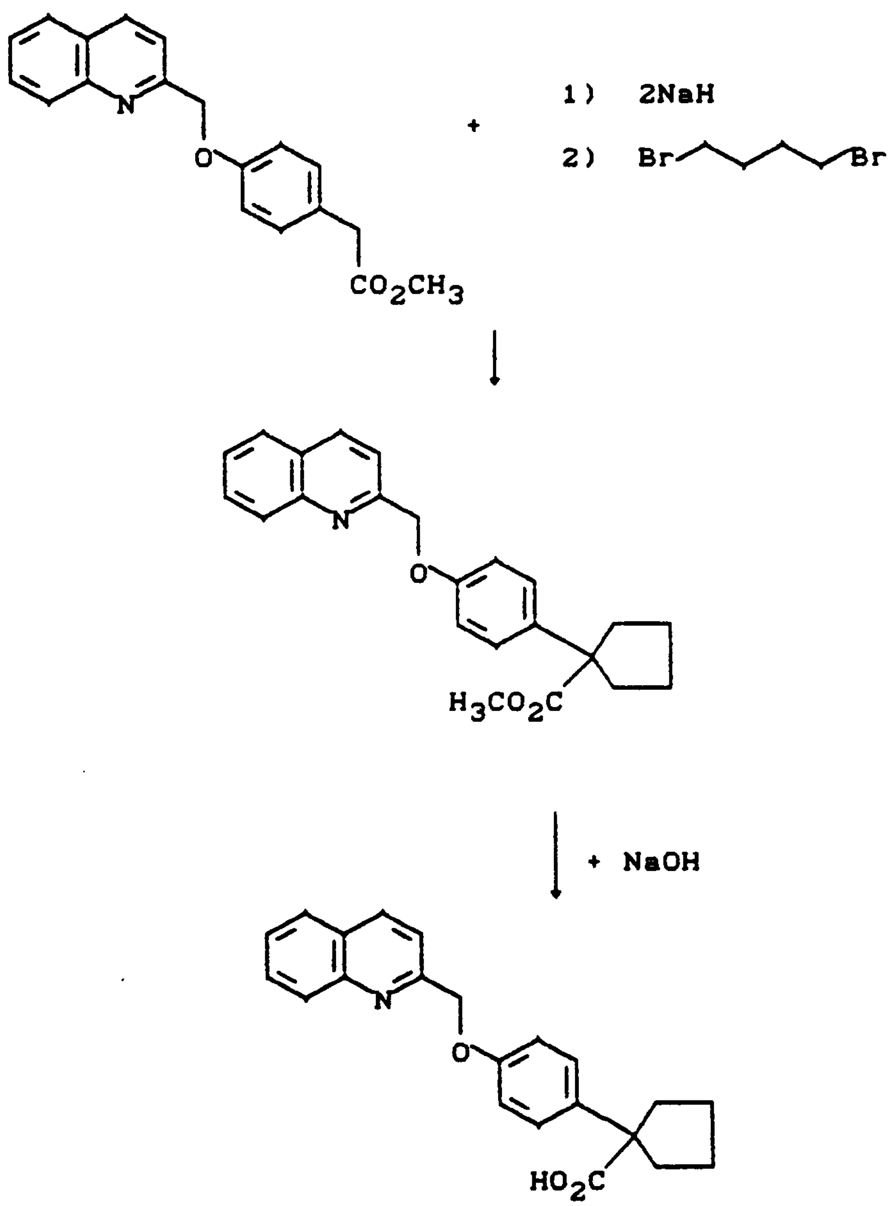

Als Lösemittel für das erfindungsgemäße Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dimethylformamid.

Als Basen eignen sich Alkalimetalle, Alkalihydride, Alkaliamide, Alkalialkoholate oder lithiumorganische Verbindungen, bevorzugt sind Alkalihydride wie Natriumhydrid oder Kaliumhydrid.

Das erfindungsgemäße Verfahren wird im allgemeinen in einem Temperaturbereich von -10°C bis +80°C, bevorzugt bei Raumtemperatur durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Im allgemeinen setzt man 2 bis 3 Mol, bevorzugt 2 Mol Base, bezogen auf 1 Mol der CH-aciden-Verbindungen der allgemeinen Formel (II) ein.

Die Dihalogen-Verbindungen der allgemeinen Formel (III) sind bekannt oder können nach bekannter Methode hergestellt werden [vgl. Beilstein 1, 120; 1(32), 740; 5(3), 981].
Beispielhaft seien genannt:
cis-1,4-Dichlor-2-buten
1,2-Bis(chlormethyl)-4,5-dimethylbenzol
3,4-Bis(chlormethyl)-2,5-dimethyl-thiophen
1,4-Dibrombutan
1,5-Dibrombutan
1,5-Dibrom-3-methyl-3-phenylpentan
1,5-Dibrom-3-methyl-pentan
Es hat sich als vorteilhaft erwiesen, das oben aufgeführte Verfahren unter Schutzgas durchzuführen.

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Salze anzusäuern. Die Säuren können dann in üblicher Weiss isoliert werden [vgl. GB 2 202 223 A1; US 4 769 461 A; EP 181 568 A2].

Die CH-aciden-Verbindungen der allgemeinen Formel (II) sind teilweise bekannt und können beispielsweise hergestellt werden, indem man
Verbindungen der allgemeinen Formel (IV)

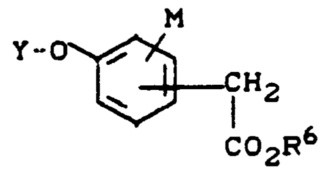

(IV)

in welcher
M und $R^6$ die oben angegebene Bedeutung haben
und
Y - für eine typische Hydroxyschutzgruppe, wie beispielsweise Benzyl oder tert.Butyl steht,
nach Abspaltung der Schutzgruppe Y nach üblicher Methode mit Halogenmethylchinolinen der Formel (V)

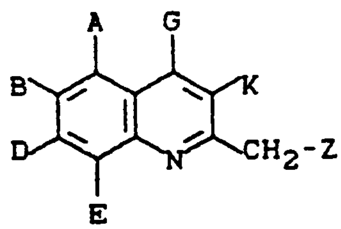

(V)

in welcher
A, B, D, E, G und K die oben angegebene Bedeutung haben, und
Z - für Halogen steht,
in inerten Lösemitteln, gegebenenfalls in Anwesenheit einer Base, verethert.

Die Abspaltung der Schutzgruppen aus den entsprechenden Ethern erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas [vgl. außerdem Th. Greene: "Protective Groups in Organic Synthesis", J. Wiley & Sons, 1981, New York].

Die Veretherung kann in inerten organischen Lösemitteln gegebenenfalls in Anwesenheit einer Base durchgeführt werden.

Lösemittel für die Veretherung können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder organische Amine (Trialkyl($C_1$-$C_6$)amine) wie Triethylamin, oder Heterocyclen wie Pyridin, Methylpiperidin, Piperidin oder Morpholin.

Es ist auch möglich, als Basen Alkalimetalle wie Natrium und deren Hydride, wie Natriumhydrid, einzusetzen.

Die Veretherung erfolgt im allgemeinen in einem Temperaturbereich von 0°C bis +150°C, bevorzugt von +10°C bis +100°C.

Die Veretherung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 Mol Halogenid, bezogen auf 1 Mol des Reaktionspartners ein. Die Base wird im allgemeinen in einer Menge von 0,5 bis 5 Mol, bevorzugt von 1 bis 3 Mol bezogen auf das Halogenid eingesetzt.

Die Verbindungen der allgemeinen Formeln (IV) und (V) sind an sich bekannt oder können nach üblicher Methode hergestellt werden [vgl. Beilstein 10, 191; C. Ferri, Reaktionen der organischen Synthese, Georg Thieme Verlag, Stuttgart 1978; Chem. Ber. 120, 649, 1987].

Die erfindungsgemäßen Verbindungen der allgemeinen Formel (I) zeigen überraschenderweise eine hohe in vitro Aktivität als Leukotriensynthesehemmer und eine starke in vivo Wirkung nach oraler Applikation.

Die erfindungsgemäßen cyclisch substituierten (Chinolin-2-yl-methoxy)phenylessigsäure-Derivate können als Wirkstoffe in Arzneimitteln eingesetzt werden. Die Stoffe können als Hemmer von enzymatischen Reaktionen im Rahmen des Arachidonsäurestoffwechsels, insbesondere der Lipoxygenase, wirken.

Sie sind somit bevorzugt zur Behandlung und Verhütung von Erkrankungen der Atemwege wie Allergien/Asthma, Bronchitis, Emphysema, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen cyclisch substituierten (Chinolin-2-yl-methoxy)phenyl-essigsäure-Derivate können sowohl in der Humanmedizin als auch in der Veterinärmedizin angewendet werden.

Die pharmakologischen Wirkdaten der erfindungsgemäßen Substanzen werden durch folgende Methode bestimmt:

Als Maß für die Lipoxygenase-Hemmung wurde die Freisetzung von Leukotrien $B_4$ ($LTB_4$) an polymorphkernigen Rattenleukozyten (PMN) nach Zugabe von Substanzen und Ca-Ionophor mittels reverse phase HPLC nach Borgeat, P. et al., Proc. Nat. Acad. Sci. 76, 2148 - 2152 (1979), bestimmt. Die in vivo-Aktivität der Verbindungen wurde mit dem Mäuseohr-Entzündungsmodell nach Young, J.M. et al., J. of Investigative Dermatology 82, 367 - 371, (1984) nachgewiesen.

Die neuen Wirkstoffe können in an sich bekannter Weise unter Verwendung inerter nichttoxischer, pharmazeutisch geeigneter Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 10 bis 70 Gew.-%, in der Zubereitung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohole-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,01 bis 5 mg/kg Körpergewicht, zur Erzielung wirksamer Ergebnisse zu verabreichen. Bei oraler Applikation beträgt die Dosierung im allgemeinen etwa 0,1 bis 200 mg/kg, vorzugsweise 1 bis 100 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und zu dem Zeitpunkt bzw. Intervall, zu welchem die Verabreichung erfolgt. So kann es in einigen Fällen ausreichend sein, mit weniger als der

vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen.

Ausgangsverbindung

Beispiel I

4-(Chinolin-2-yl-methoxy)phenylessigsäuremethylester

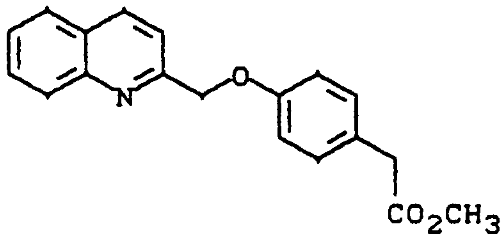

200 g (1,2 mol) 4-Hydroxyphenylessigsäuremethylester und 166 g (1,2 mol) Kaliumcarbonat werden in 2 l Dimethylformamid 1 h bei 25°C gerührt. Nach Zugabe von 214 g (1,2 mol) 2-Chlormethylchinolin wird 15 h auf 50°C erwärmt. Nach Einengen im Vakuum wird der Rückstand zwischen Wasser und Essigester verteilt, die organische Phase über Natriumsulfat getrocknet und eingeengt. Das verbleibende Produkt wird aus Methanol umkristallisiert.
Ausbeute: 293 g (79% der Theorie)
Festpunkt: 71 - 73°C

Herstellungsbeispiele (Formel I)

Beispiel 1

1-[4-(Chinolin-2-yl-methoxy)phenyl]cyclopentancarbonsäuremethylester

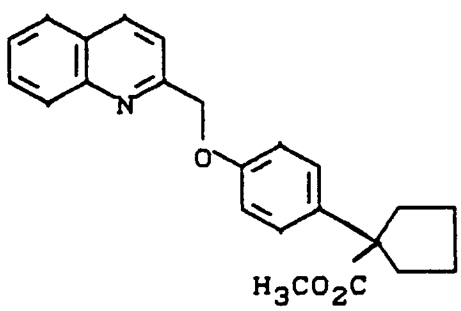

10 g (32,5 mmol) der Verbindung aus Beispiel I und 3,88 ml (32,5 mmol) 1,4-Dibrombutan werden unter Schutzgas in 100 ml Dimethylformamid gelöst und portionsweise 1,95 g (65 mmol) Natriumhydrid (80%ig) zugegeben. Es wird 15 h bei Raumtemperatur gerührt, anschließend auf 200 ml Eiswasser gegeben und abgesaugt. Das Rohprodukt wird an Kieselgel 60 mit Methylenchlorid/Methanol (100:2) chromatographiert.
Ausbeute: 5,47 g (46,6% der Theorie)
Fp.: 136°C (Methanol)
Die in Tabelle 1 aufgeführten Verbindungen wurden in Analogie zur Vorschrift des Beispiels 2 hergestellt.

Ar* = 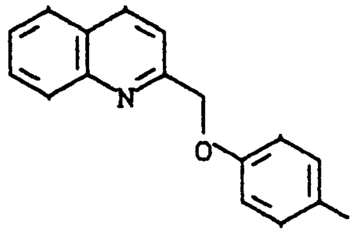

Tabelle 1

| Bsp.-Nr. | 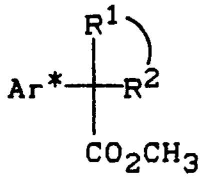 | Fp.°C | Ausbeute % |
|---|---|---|---|
| 2 | 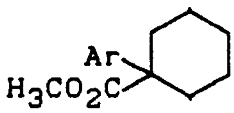 | 85 | 35 |
| 3 | 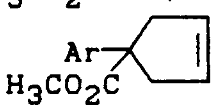 | 124-126 | 15 |
| 4 | 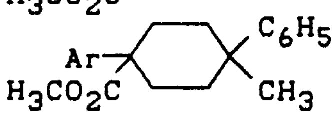 | 143 | 50 |
| 5 | 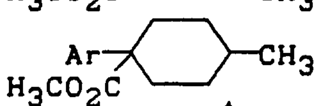 | 148 | 28 |
| 6 | 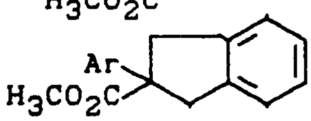 | 142 | 7,5 |
| 7 | 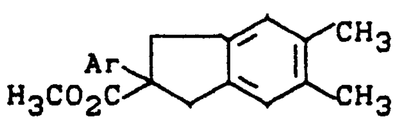 | 177 | 25 |
| 8 | 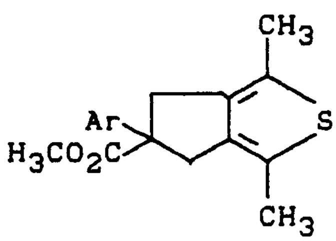 | 115-118 | 6,5 |

Beispiel 9

1-[4-(Chinolin-2-yl-methoxy)phenyl]cyclopentancarbonsäure

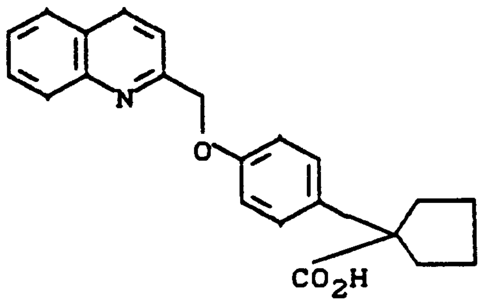

5 g (13,8 mmol) der Verbindung aus Beispiel 1 werden in 50 ml Dioxan und 15 ml 2 normaler Natronlauge 15 h unter Rückfluß erwärmt. Nach Abkühlen wird mit Salzsäure neutralisiert und einrotiert. Das Produkt wird in Wasser verrührt, abgesaugt, getrocknet und aus Methanol umkristallisiert.
Ausbeute: 4,38 g (91,5% der Theorie)
Fp.: 195 ° C (Methanol)
Die in Tabelle 2 aufgeführten Verbindungen wurden in Analogie zur Vorschrift des Beispiels 9 hergestellt.

<u>Tabelle 2</u>

AR* = 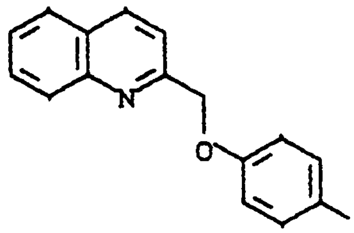

| Bsp.-Nr. | 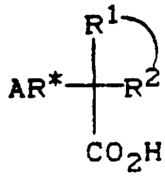 | Fp.° C | Ausbeute % |
|---|---|---|---|
| 10 | 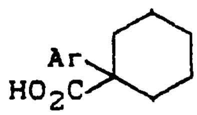 | 170 | 85 |
| 11 | 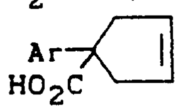 | 194 | 97 |
| 12 | 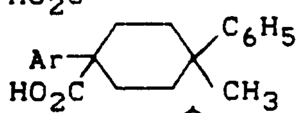 | 192 | 75 |
| 13 | 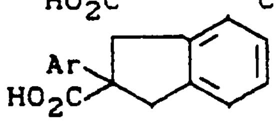 | 207 | 72 |
| 14 | 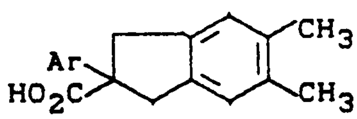 | >260 | 46 |
| 15 | 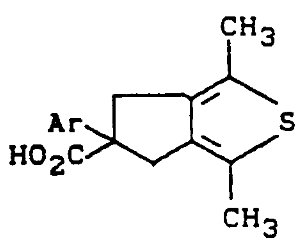 | 226 | 81 |

**Patentansprüche**

**Patentansprüche für folgende Vertragsstaaten : AT, BE, CH, DE, DK, FR, GB, GR, IT, LI, LU, NL, SE**

1. Cyclisch substituierte (Chinolin-2-yl-methoxy)phenylessigsäure-Derivate der allgemeinen Formel

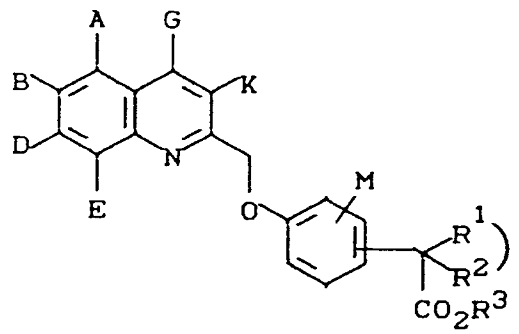

(I)

in welcher

A, B, D, E, G, K und M gleich oder verschieden sind und
- für Wasserstoff, Hydroxy, Halogen, Trifluormethyl, Trifluormethoxy oder Carboxy stehen,
- für geradkettiges oder verzweigtes Alkyl mit bis zu 10 Kohlenstoffatomen stehen, das gegebenenfalls durch Hydroxy oder Halogen substituiert ist,
- für geradkettiges oder verzweigtes Alkoxy oder Alkoxycarbonyl mit bis zu 10 Kohlenstoffatomen stehen, oder
- für Aryl mit 6 bis 10 Kohlenstoffatomen stehen, das gegebenenfalls durch Halogen, Nitro, Cyano oder durch geradkettiges oder verzweigtes Alkyl oder Alkoxy mit bis zu 8 Kohlenstoffatomen substituiert ist,

$R^1$ und $R^2$ gemeinsam einen 4- bis 8-gliedrigen, gesättigtigten oder ungesättigten Carbocyclus bilden, der gegebenenfalls bis zu 3-fach gleich oder verschieden durch geradkettiges oder verzweigtes Alkyl oder Alkoxy mit jeweils bis zu 8 Kohlenstoffatomen, Halogen, Hydroxy oder Aryl mit 6 bis 10 Kohlenstoffatomen substituiert ist, oder

$R^1$ und $R^2$ gemeinsam mit dem Kohlenstoffatom für einen Rest der Formel

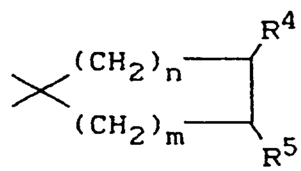

stehen,

worin

m und n gleich oder verschieden sind und eine Zahl 0, 1, 2, 3 oder 4 bedeuten,

und

$R^4$ und $R^5$ gemeinsam Aryl mit 6 bis 10 Kohlenstoffatomen oder einen 5- bis 7-gliedrigen, gesättigten oder ungesättigten Heterocyclus mit bis zu 3 Heteroatomen aus der Reihe Stickstoff, Schwefel oder Sauerstoff bilden, die gegebenenfalls durch geradkettiges oder verzweigtes Alkyl oder Alkoxy mit jeweils bis zu 8 Kohlenstoffatomen, Halogen oder Aryl mit 6 bis 10 Kohlenstoffatomen substituiert sind,

$R^3$ - für Wasserstoff, geradkettiges oder verzweigtes Alkyl mit bis zu 8 Kohlenstoffatomen oder Phenyl steht

und deren Salze.

2. Cyclisch substituierte (Chinolin-2-yl-methoxy)phenylessigsäure-Derivate der Formel nach Anspruch 1, worin

A, B, D, E, G, K und M gleich oder verschieden sind und
- für Wasserstoff, Fluor, Chlor, Trifluormethoxy oder Carboxy stehen,
- für geradkettiges oder verzweigtes Alkyl mit bis zu 8 Kohlenstoffatomen stehen, das gegebenenfalls durch Hydroxy, Fluor, Chlor oder Brom substituiert ist,
- für geradkettiges oder verzweigtes Alkoxy oder Alkoxycarbonyl mit bis zu 8 Kohlenstoffatomen stehen, oder
- für Phenyl stehen, das gegebenenfalls durch Fluor, Chlor, Brom, Nitro, Cyano oder durch geradkettiges oder verzweigtes Alkyl oder Alkoxy mit bis zu 6 Kohlenstoffatomen substituiert ist,

$R^1$ und $R^2$ gemeinsam für Cyclopropyl, Cyclobutyl, Cyclopentyl, Cyclohexyl, Cycloheptyl, Cyclopentenyl, Cyclohexenyl oder Cycloheptenyl stehen, die gegebenenfalls bis zu 2-fach gleich oder verschieden durch geradkettiges oder verzweigtes Alkyl mit bis zu 6 Kohlenstoffatomen oder Phenyl substituiert sind, oder

$R^1$ und $R^2$ gemeinsam mit dem Kohlenstoffatom für einen Rest der Formel

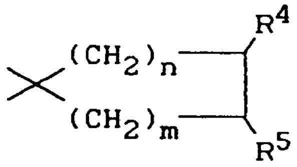

stehen,
worin

m und n gleich oder verschieden sind und eine Zahl 0, 1, 2 oder 3 bedeuten,

$R^4$ und $R^5$ gemeinsam Phenyl, Furanyl, Thienyl, Pyridyl oder Pyrryl bedeuten, die gegebenenfalls durch geradkettiges oder verzweigtes Alkyl oder Alkoxy mit jeweils bis zu 6 Kohlenstoffatomen, Fluor, Chlor, Brom oder Phenyl substituiert sind,

$R^3$ - für Wasserstoff, geradkettiges oder verzweigtes Alkyl mit bis zu 8 Kohlenstoffatomen oder Phenyl steht

und deren Salze.

3. Cyclisch substituierte (Chinolin-2-yl-methoxy)phenylessigsäure-Derivate der Formel nach Anspruch 1 worin

A, B, D, E, G, K und M gleich oder verschieden sind und
- für Wasserstoff, Fluor, Chlor, geradkettiges oder verzweigtes Alkyl oder Alkoxy mit bis zu 6 Kohlenstoffatomen stehen,

$R^1$ und $R^2$ gemeinsam für Cyclopentyl, Cyclopentenyl, Cyclohexyl, Cyclohexenyl, Cycloheptyl oder Cycloheptenyl stehen, die gegebenenfalls durch geradkettiges oder verzweigtes Alkyl mit bis zu 4 Kohlenstoffatomen und/oder Phenyl substituiert sind, oder

$R^1$ und $R^2$ gemeinsam mit dem Kohlenstoffatom für einen Rest der Formel

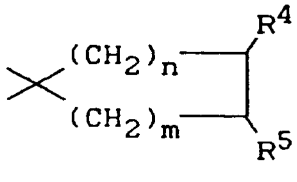

stehen,
worin

m und n gleich oder verschieden sind und eine Zahl 1 oder 2 bedeuten,

$R^4$ und $R^5$ gemeinsam Phenyl, Thienyl, Pyridyl oder Pyrryl bedeuten, die gegebenenfalls durch geradkettiges oder verzweigtes Alkyl mit bis zu 4 Kohlenstoffatomen oder Phenyl substituiert sind,

$R^3$ - für Wasserstoff oder geradkettiges oder verzweigtes Alkyl mit bis zu 6 Kohlenstoffatomen steht

und deren Salze.

**4.** Cyclisch substituierte (Chinolin-2-yl-methoxy)phenylessigsäure-Derivate nach Anspruch 1 - 3 zur Bekämpfung von Krankheiten.

**5.** Verfahren zur Herstellung von cyclisch substituierten (Chinolin-2-yl-methoxy)phenylessigsäure-Derivaten nach Anspruch 1 - 3
und deren Salze, dadurch gekennzeichnet, daß man CH-acide-Verbindungen der allgemeinen Formel

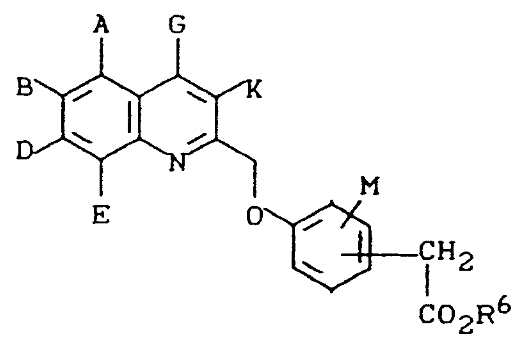

(II)

in welcher
A, B, D, E, G, K und M die oben angegebene Bedeutung haben,
und
$R^6$ - die oben angegebene Bedeutung von $R^3$ hat aber nicht für Wasserstoff steht,
mit Dihalogen-Verbindungen der allgemeinen Formel (III)

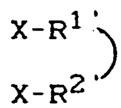

(III)

in welcher
$R^{1'}$ und $R^{2'}$ die oben angegebene Bedeutung von $R^1$ und $R^2$ haben, aber in der offenkettigen Form vorliegen,
und
X - für Halogen steht,
in inerten Lösemitteln, gegebenenfalls in Anwesenheit einer Base umsetzt, und dann im Fall der Sauren die Ester verseift.

**6.** Arzneimittel enthaltend cyclisch substituierte (Chinolin-2-yl-methoxy)phenylessigsäure-Derivate nach Anspruch 1 bis 3.

**7.** Verfahren zur Herstellung eines Arzneimittels nach Anspruch 6, dadurch gekennzeichnet, daß man Verbindungen nach Anspruch 1-3 gegebenenfalls mit Hilfe von üblichen Hilfs- und Trägerstoffen in eine geeignete Applikationsform bringt.

**8.** Verwendung von cyclisch substituierten (Chinolin-2-yl-methoxy)phenylessigsäure-Derivaten nach Anspruch 1 - 3 zur Herstellung von Arzneimitteln.

9. Verwendung von cyclisch substituierten (Chinolin-2-yl-methoxy)phenylessigsäure-Derivaten nach Anspruch 1 - 3 zur Herstellung von Lipoxygenasehemmern.

**Patentansprüche für folgenden Vertragsstaat : ES**

1. Verfahren zur Herstellung von cyclisch substituierten (Chinolin-2-yl-methoxy)phenylessigsäure-Derivaten der allgemeinen Formel

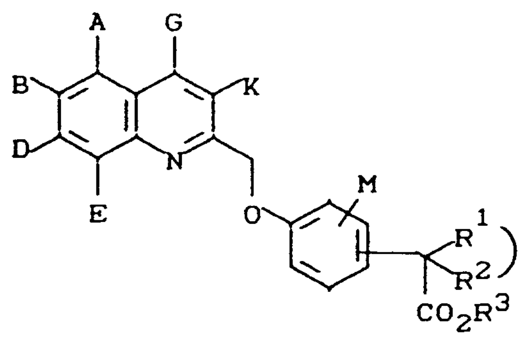

(I)

in welcher

A, B, D, E, G, K und M gleich oder verschieden sind und
- für Wasserstoff, Hydroxy, Halogen, Trifluormethyl, Trifluormethoxy oder Carboxy stehen,
- für geradkettiges oder verzweigtes Alkyl mit bis zu 10 Kohlenstoffatomen stehen, das gegebenenfalls durch Hydroxy oder Halogen substituiert ist,
- für geradkettiges oder verzweigtes Alkoxy oder Alkoxycarbonyl mit bis zu 10 Kohlenstoffatomen stehen, oder
- für Aryl mit 6 bis 10 Kohlenstoffatomen stehen, das gegebenenfalls durch Halogen, Nitro, Cyano oder durch geradkettiges oder verzweigtes Alkyl oder Alkoxy mit bis zu 8 Kohlenstoffatomen substituiert ist,

$R^1$ und $R^2$ gemeinsam einen 4- bis 8-gliedrigen, gesättigtigten oder ungesättigten Carbocyclus bilden, der gegebenenfalls bis zu 3-fach gleich oder verschieden durch geradkettiges oder verzweigtes Alkyl oder Alkoxy mit jeweils bis zu 8 Kohlenstoffatomen, Halogen, Hydroxy oder Aryl mit 6 bis 10 Kohlenstoffatomen substituiert ist, oder

$R^1$ und $R^2$ gemeinsam mit dem Kohlenstoffatom für einen Rest der Formel

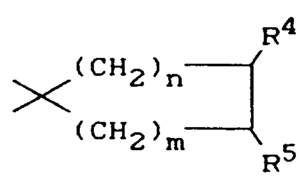

stehen,

worin

m und n gleich oder verschieden sind und eine Zahl 0, 1, 2, 3 oder 4 bedeuten,

und

$R^4$ und $R^5$ gemeinsam Aryl mit 6 bis 10 Kohlenstoffatomen oder einen 5- bis 7-gliedrigen, gesättigten oder ungesättigten Heterocyclus mit bis zu 3 Heteroatomen aus der Reihe Stickstoff, Schwefel oder Sauerstoff bilden, die gegebenenfalls durch geradkettiges oder verzweigtes Alkyl oder Alkoxy mit jeweils bis zu 8 Kohlenstoffatomen, Halogen oder Aryl mit 6 bis 10 Kohlenstoffatomen substituiert sind,

$R^3$ - für Wasserstoff, geradkettiges oder verzweigtes Alkyl mit bis zu 8 Kohlenstoffatomen oder Phenyl steht

und deren Salze, dadurch gekennzeichnet, daß man CH-acide-Verbindungen der allgemeinen Formel

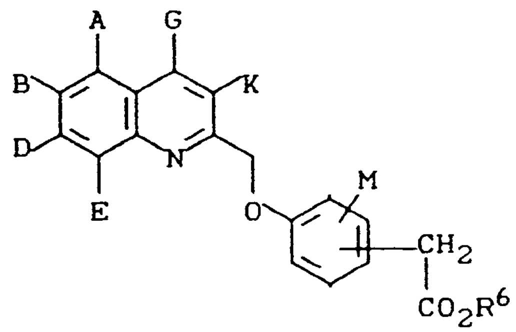

(II)

in welcher

A, B, D, E, G, K und M die oben angegebene Bedeutung haben,

und

$R^6$ - die oben angegebene Bedeutung von $R^3$ hat aber nicht für Wasserstoff steht,

mit Dihalogen-Verbindungen der allgemeinen Formel (III)

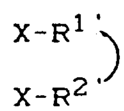

(III)

in welcher

$R^{1'}$ und $R^{2'}$ die oben angegebene Bedeutung von $R^1$ und $R^2$ haben, aber in der offenkettigen Form vorliegen,

und

X - für Halogen steht,

in inerten Lösemitteln, gegebenenfalls in Anwesenheit einer Base umsetzt, und dann im Fall der Säuren die Ester verseift.

**Claims**

**Claims for the following Contracting States : AT, BE, CH, DE, DR, FR, GB, GR, IT, LI, LU, NL, SE**

1. (Quinolin-2-yl-methoxy)phenylacetic acid derivatives containing cyclic substituents, of the general formula

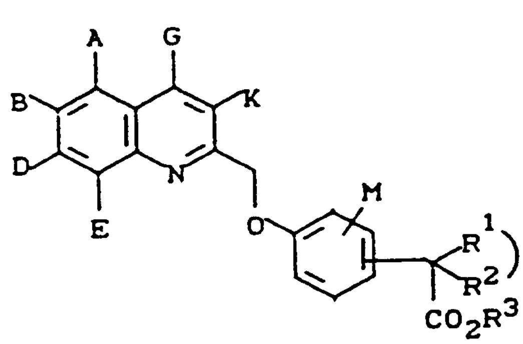

(I)

in which

A, B, D, E, G, K and M are identical or different and

- represent hydrogen, hydroxyl, halogen, trifluoromethyl, trifluoromethoxy or carboxyl,
- represent straight-chain or branched alkyl having up to 10 carbon atoms, which is optionally substituted by hydroxyl or halogen,
- represent straight-chain or branched alkoxy or alkoxycarbonyl having up to 10 carbon atoms, or
- represent aryl having 6 to 10 carbon atoms, which is optionally substituted by halogen, nitro, cyano or by straight-chain or branched alkyl or alkoxy having up to 8 carbon atoms,

$R^1$ and $R^2$ together form a 4- to 8-membered, saturated or unsaturated carbocyclic ring, which is optionally substituted by up to 3 identical or different substituents from the group comprising straight-chain or branched alkyl or alkoxy having in each case up to 8 carbon atoms, halogen, hydroxyl and aryl having 6 to 10 carbon atoms, or

$R^1$ and $R^2$, together with the carbon atom, represent a radical of the formula

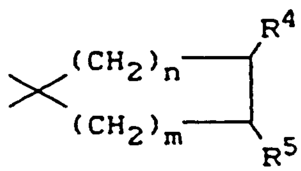

wherein

m and n are identical or different and denote the number 0, 1, 2, 3 or 4,

and

$R^4$ and $R^5$ together form aryl having 6 to 10 carbon atoms or a 5- to 7-membered saturated or unsaturated heterocyclic radical having up to 3 heteroatoms from the series comprising nitrogen, sulphur or oxygen, which are optionally substituted by straight-chain or branched alkyl or alkoxy having in each case up to 8 carbon atoms, halogen or aryl having 6 to 10 carbon atoms, and

$R^3$ - represents hydrogen, straight-chain or branched alkyl having up to 8 carbon atoms or phenyl,

and salts thereof.

**2.** (Quinolin-2-yl-methoxy)phenylacetic acid derivatives containing cyclic substituents, of the formula according to Claim 1,

wherein

A, B, D, E, G, K and M are identical or different and

- represent hydrogen, fluorine, chlorine, trifluoromethoxy or carboxyl,
- represent straight-chain or branched alkyl having up to 8 carbon atoms, which is optionally substituted by hydroxyl, fluorine, chlorine or bromine,
- represent straight-chain or branched alkoxy or alkoxycarbonyl having up to 8 carbon atoms, or
- represent phenyl, which is optionally substituted by fluorine, chlorine, bromine, nitro, cyano or by straight-chain or branched alkyl or alkoxy having up to 6 carbon atoms,

$R^1$ and $R^2$ together represent cyclopropyl, cyclobutyl, cyclopentyl, cyclohexyl, cycloheptyl, cyclopentenyl, cyclohexenyl or cycloheptenyl, which are optionally substituted by up to 2 identical or different substituents from the group comprising straight-chain or branched alkyl having up to 6 carbon atoms or phenyl, or

$R^1$ and $R^2$, together with the carbon atom, represent a radical of the formula

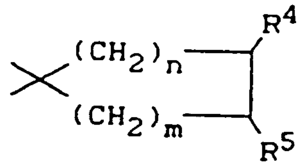

wherein

m and n — are identical or different and denote the number 0, 1, 2 or 3,

$R^4$ and $R^5$ — together denote phenyl, furanyl, thienyl, pyridyl or pyrryl, which are optionally substituted by straight-chain or branched alkyl or alkoxy having in each case up to 6 carbon atoms, fluorine, chlorine, bromine or phenyl, and

$R^3$ — - represents hydrogen, straight chain or branched alkyl having up to 8 carbon atoms or phenyl,

and salts thereof.

**3.** (Quinolin-2-yl-methoxy)phenylacetic acid derivatives containing cyclic substituents, of the formula according to Claim 1,
wherein

A, B, D, E, G, K and M — are identical or different and
- represent hydrogen, fluorine, chlorine, straight-chain or branched alkyl or alkoxy having up to 6 carbon atoms,

$R^1$ and $R^2$ — together represent cyclopentyl, cyclopentenyl, cyclohexyl, cyclohexenyl, cycloheptyl or cycloheptenyl, which are optionally substituted by straight-chain or branched alkyl having up to 4 carbon atoms and/or phenyl, or

$R^1$ and $R^2$ together with the carbon atom, represent a radical of the formula

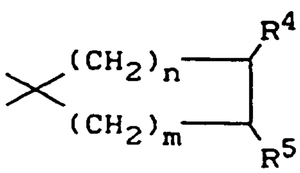

m and n are identical or different and denote the number 1 or 2,

$R^4$ and $R^5$ — together denote phenyl, thienyl, pyridyl or pyrryl, which are optionally substituted by straight-chain or branched alkyl having up to 4 carbon atoms or phenyl, and

$R^3$ — - represents hydrogen or straight-chain or branched alkyl having up to 6 carbon atoms,

and salts thereof.

**4.** (Quinolin-2-yl-methoxy)phenylacetic acid derivatives containing cyclic substituents, according to Claims 1-3, for combating diseases.

**5.** Process for the preparation of (quinolin-2-yl-methoxy)phenylacetic acid derivatives containing cyclic substituents, according to claims 1-3
and salts thereof, characterized in that acidic CH compounds of the general formula

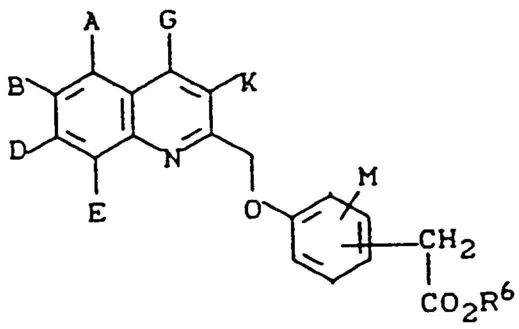

(II)

in which

A, B, D, E, G, K and M have the abovementioned meaning, and

$R^6$ - has the abovementioned meaning of $R^3$ but does not represent hydrogen, are reacted with dihalogeno compounds of the general formula (III)

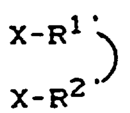

(III)

in which

$R^{1'}$ and $R^{2'}$ have the abovementioned meaning of $R^1$ and $R^2$, but are in the open-chain form,

and

X - represents halogen,

in inert solvents, if appropriate in the presence of a base, and in the case of the acids the esters are then hydrolysed.

6. Medicaments containing (quinolin-2-yl-methoxy)phenylacetic acid derivatives containing cyclic substituents, according to Claims 1 to 3.

7. Process for the preparation of a medicament according to Claim 6, characterized in that compounds according to Claims 1-3 are brought into a suitable administration form, if appropriate with the aid of customary auxiliaries and excipients.

8. Use of (quinolin-2-yl-methoxy)phenylacetic acid derivatives containing cyclic substituents, according to Claims 1-3, for the preparation of medicaments.

9. Use of (quinolin-2-yl-methoxy)phenylacetic acid derivatives containing cyclic substituents, according to Claims 1-3, for the preparation of lipoxygenase inhibitors.

**Claims for the following Contracting State : ES**

1. Process for the preparation of (quinolin-2-yl-methoxy)phenylacetic acid derivatives containing cyclic substituents, of the general formula

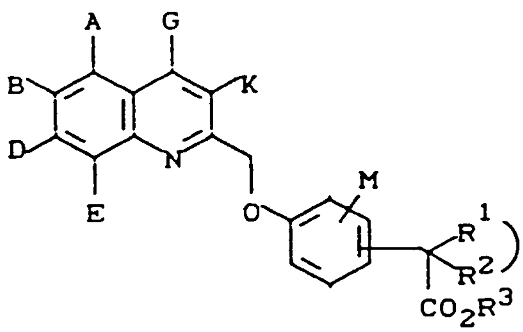

(I)

in which

A, B, D, E, G, K and M are identical or different and
- represent hydrogen, hydroxyl, halogen, trifluoromethyl, trifluoromethoxy or carboxyl,
- represent straight-chain or branched alkyl having up to 10 carbon atoms, which is optionally substituted by hydroxyl or halogen,
- represent straight-chain or branched alkoxy or alkoxycarbonyl having up to 10 carbon atoms, or
- represent aryl having 6 to 10 carbon atoms, which is optionally substituted by halogen, nitro, cyano or by straight-chain or branched alkyl or alkoxy having up to 8 carbon atoms,

$R^1$ and $R^2$ together form a 4- to 8-membered, saturated or unsaturated carbocyclic ring, which is optionally substituted by up to 3 identical or different substituents from the group comprising straight-chain or branched alkyl or alkoxy having in each case up to 8 carbon atoms, halogen, hydroxyl and aryl having 6 to 10 carbon atoms, or

$R^1$ and $R^2$, together with the carbon atom, represent a radical of the formula

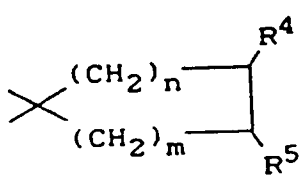

wherein

m and n are identical or different and denote the number 0, 1, 2, 3 or 4,

and

$R^4$ and $R^5$ together form aryl having 6 to 10 carbon atoms or a 5- to 7-membered saturated or unsaturated heterocyclic radical having up to 3 heteroatoms from the series comprising nitrogen, sulphur or oxygen, which are optionally substituted by straight-chain or branched alkyl or alkoxy having in each case up to 8 carbon atoms, halogen or aryl having 6 to 10 carbon atoms, and

$R^3$ - represents hydrogen, straight-chain or branched alkyl having up to 8 carbon atoms or phenyl,

and salts thereof, characterized in that acidic CH compounds of the general formula

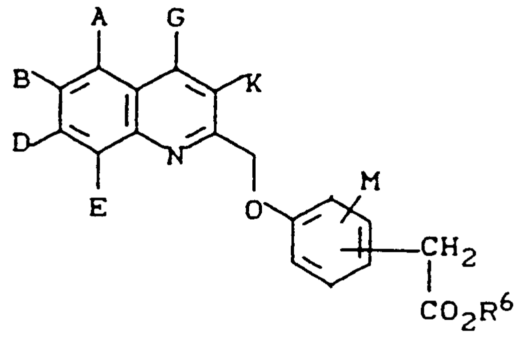

(II)

in which
A, B, D, E, G, K and M have the abovementioned meaning, and
$R^6$ - has the abovementioned meaning of $R^3$ but does not represent hydrogen,
are reacted with dihalogeno compounds of the general formula (III)

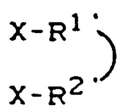

(III)

in which
$R^{1'}$ and $R^{2'}$ have the abovementioned meaning of $R^1$ and $R^2$, but are in the open-chain form,
and
X - represents halogen,
in inert solvents, if appropriate in the presence of a base, and in the case of the acids the esters are then hydrolysed.

**Revendications**
**Revendications pour les Etats contractants suivants : AT, BE, CH, DE, DK, FR, GB, GR, IT, LI, LU, NL, SE**

**1.** Dérivés de l'acide (quinoléin-2-yl-méthoxy)phénylacétique substitués par un radical cyclique, répondant à la formule générale

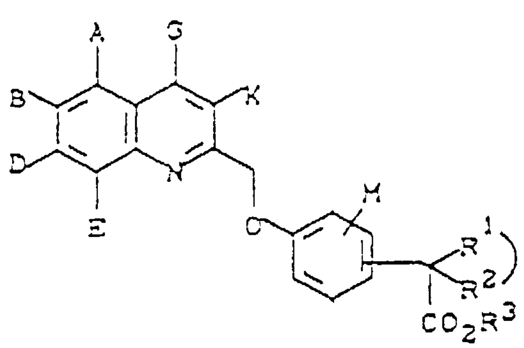

(I)

dans laquelle
A, B, D, E, G, K et M sont identiques ou différents et représentent
- un atome d'hydrogène, un groupe hydroxyle, un atome d'halogène, un groupe trifluorométhyle, un groupe trifluorométhoxy ou un groupe carboxyle,

- un groupe alkyle à chaîne droite ou ramifiée contenant jusqu'à 10 atomes de carbone, qui est éventuellement substitué une ou plusieurs fois par un groupe hydroxyle ou par un atome à'halogène,
- un groupe alcoxy ou un groupe alcoxycarbonyle à chaîne droite ou ramifiée contenant jusqu'à 10 atomes de carbone, ou
- représentent un groupe aryle contenant de 6 à 10 atones de carbone, qui est éventuellement substitué une ou plusieurs fois par un atome d'halogène, par un groupe nitro, par un groupe cyano ou encore par un groupe alkyle ou par un groupe alcoxy à chaîne droite ou ramifiée contenant jusqu'à 8 atomes de carbone,

$R^1$ et $R^2$ forment ensemble un radical carbocyclique saturé ou insature contenant de 4 à 8 chaînons qui est éventuellement substitué jusqu'à 3 fois de manière identique ou différente par un groupe alkyle ou par un groupe alcoxy à chaîne droite ou ramifiée contenant chacun jusqu'à 8 atomes de carbone, par un atome d'halogène, par un groupe hydroxyle ou par un groupe aryle contenant de 6 à 10 atomes de carbone, ou

$R^1$ et $R^2$ représentent, conjointement avec l'atome de carbone, un radical de formule

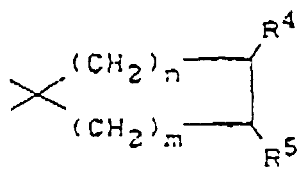

dans laquelle

m et n sont identiques ou différents et désignent le nombre or 1 2, 3 ou 4,

et

$R^4$ et $R^5$ forment ensemble un groupe aryle contenant de 6 à 10 atomes de carbone ou un radical hétérocyclique penta- à heptagonal, saturé ou insaturé, contenant jusqu'à 3 hétéroatomes choisis parmi la série comprenant un atome d'azote, un atome de soufre ou un atome d'oxygène, qui sont éventuellement substitués une ou plusieurs fois par un groupe alkyle ou par un groupe alcoxy à chaîne droite ou ramifiée contenant chacun jusqu'à 8 atomes de carbone, par un atome d'halogène ou par un groupe aryle contenant de 6 à 10 atomes de carbone,

$R^3$ représente un atome d'hydrogène, un groupe alkyle à chaîne droite ou ramifiée contenant jusqu'à 8 atomes de carbone ou encore un groupe phényle,

ainsi que leurs sels.

**2.** Dérivés de l'acide (quinoléin-2-ylméthoxy)phénylacétique substitués par un radical cyclique, répondant à la formule selon la revendication 1,

dans laquelle

A, B, D, E, G, K et M sont identiques ou différents et représentent

- un atome d'hydrogène, un atome de fluor, un atome de chlore, un groupe trifluorométhoxy ou un groupe carboxyle,
- un groupe alkyle à chaîne droite ou ramifiée contenant jusqu'à 8 atomes de carbone, qui est éventuellement substitué une ou plusieurs fois par un groupe hydroxyle, par un atome de fluor, par un atome de chlore ou par un atome de brome,
- un groupe alcoxy ou un groupe alcoxycarbonyle à chaîne droite ou ramifiée contenant jusqu'à 8 atomes de carbone, ou
- représentent un groupe phényle qui est éventuellement substitué une ou plusieurs fois par un atome de fluor, par un atome de chlore, par un atome de brome, par un groupe nitro, par un groupe cyano ou encore par un groupe alkyle ou par un groupe alcoxy à chaîne droite ou ramifiée contenant jusqu'à 6 atomes de carbone,

$R^1$ et $R^2$ représentent ensemble un groupe cyclopropyle, un groupe cyclobutyle, un

groupe cyclopentyle, un groupe cyclohexyle, un groupe cycloheptyle, un groupe cyclopentényle, un groupe cyclohexényle ou un groupe cycloheptényle, qui sont éventuellement substitués jusqu'à deux fois de manière identique ou différente par un groupe alkyle à chaîne droite ou ramifiée contenant jusqu'à 6 atomes de carbone ou encore par un groupe phényle, ou

$R^1$ et $R^2$ représentent, conjointement avec l'atome de carbone, un radical de formule

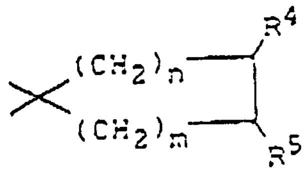

dans laquelle

m et n sont identiques ou différents et désignent le nombre 0, 1, 2 ou 3,

$R^4$ et $R^5$ représentent ensemble un groupe phényle, un groupe furanyle, un groupe thiényle, un groupe pyridyle ou un groupe pyrryle, qui sont éventuellement substitués une ou plusieurs fois par un groupe alkyle ou un groupe alcoxy à chaîne droite ou ramifiée contenant chacun jusqu'à 6 atomes de carbone, par un atome de fluor, par un atome de chlore, par un atome de brome ou par un groupe phényle,

$R^3$ représente un atome d'hydrogène, un groupe alkyle à chaîne droite ou ramifiée contenant jusqu'à 8 atomes de carbone ou encore un groupe phényle,

ainsi que leurs sels.

**3.** Dérivés de l'acide (quinoléin-2-ylméthoxy)phénylacétique substitués par un radical cyclique, répondant à la formule selon la revendication 1,

dans laquelle

A, B, D, E, G, K et M sont identiques ou différents et représentent

- un atome d'hydrogène, un atome de fluor, un atome de chlore, un groupe alkyle ou un groupe alcoxy à chaîne droite ou ramifiée contenant jusqu'à 6 atomes de carbone,

$R^1$ et $R^2$ représentent ensemble un groupe cyclopentyle, un groupe cyclopentényle, un groupe cyclohexyle, un groupe cyclohexényle, un groupe cycloheptyle ou un groupe cycloheptényle, qui sont éventuellement substitués une ou plusieurs fois par un groupe alkyle à chaîne droite ou ramifiée contenant jusqu'à 4 atomes de carbone et/ou par un groupe phényle, ou

$R^1$ et $R^2$ représentent, conjointement avec l'atome de carbone, un radical de formule

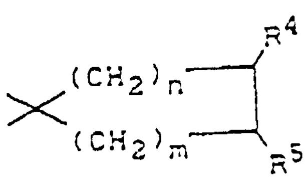

dans laquelle

m et n sont identiques ou différents et représentent le nombre 1 ou 2,

$R^4$ et $R^5$ représentent ensemble un groupe phényle, un groupe thiényle, un groupe pyridyle ou un groupe pyrryle, qui sont éventuellement substitués une ou plusieurs fois par un groupe alkyle à chaîne droite ou ramifiée contenant jusqu'à 4 atomes de carbone, ou encore par un groupe phényle,

$R^3$ représente un atome d'hydrogène ou un groupe alkyle à chaîne droite ou ramifiée contenant jusqu'à 6 atomes de carbone,

ainsi que leurs sels.

4. Dérivés de l'acide (quinoléin-2-ylméthoxy)phénylacétique substitués par un radical cyclique, selon les revendications 1-3, pour lutter contre des maladies.

5. Procédé pour la préparation de dérivés de l'acide (quinoléin-2-ylméthoxy)phénylacétique substitués par un radical cyclique, selon les revendications 1-3,
et de leurs sels, caractérisé en ce qu'on fait réagir des composés CH-acides répondant à la formule générale

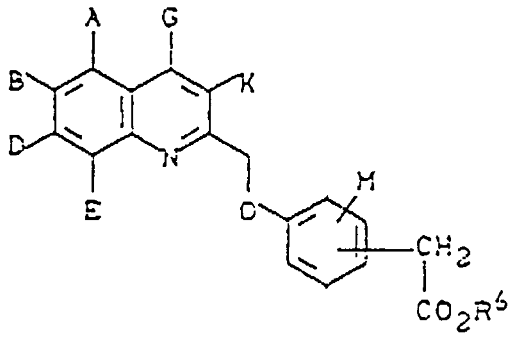

(II)

dans laquelle
A, B, D, E, G, K et M ont la signification indiquée ci-dessus,
et

$R^6$ a la signification de $R^3$ indiquée ci-dessus, mais ne représente pas un atome d'hydrogène,

avec des composés dihalogénés répondant à la formule générale (III)

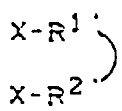

(III)

dans laquelle

$R^{1'}$ et $R^{2'}$ ont la signification indiquée ci-dessus pour $R^1$ et $R^2$, mais sont présents sous la forme d'une chaîne ouverte,

et

X représente un atome d'halogène,

dans des solvants inertes, éventuellement en présence d'une base, et ensuite, on saponifie les esters dans le cas des acides.

6. Médicaments contenant des dérivés de l'acide (quinoléin-2-ylméthoxy)phénylacétique substitués par un radical cyclique selon les revendications 1 à 3.

7. Procédé pour la préparation d'un médicament selon la revendication 6, caractérisé en ce qu'on amène à une forme d'application appropriée des composés selon les revendications 1-3, éventuellement à l'aide de substances auxiliaires et de support habituelles.

8. Utilisation de dérivés de l'acide (quinoléin-2-yl-méthoxy)phénylacétique substitués par un radical cyclique selon les revendications 1-3, pour la préparation de médicaments.

9. Utilisation de dérivés de l'acide (quinoléin-2-ylméthoxy)phénylacétique substitués par un radical cyclique selon les revendications 1-3, pour la préparation d'inhibiteurs de la lipoxygénase.

**Revendications pour l'Etat contractant suivant : ES**

1. Procédé pour la préparation de dérivés de l'acide (quinoléin-2-yl-méthoxy)phénylacétique substitués par un radical cyclique, répondant à la formule générale

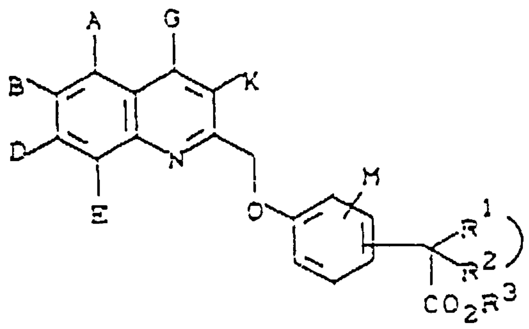

(I)

dans laquelle

A, B, D, E, G, K et M sont identiques ou différents et représentent

- un atome d'hydrogène, un groupe hydroxyle, un atome d'halogène, un groupe trifluorométhyle, un groupe trifluorométhoxy ou un groupe carboxyle,
- un groupe alkyle a chaîne droite ou ramifiée contenant jusqu'à 10 atomes de carbone, qui est éventuellement substitué une ou plusieurs fois par un groupe hydroxyle ou par un atome d'halogène,
- un groupe alcoxy ou un groupe alcoxycarbonyle à chaîne droite ou ramifiée contenant jusqu'à 10 atomes de carbone, ou
- représentent un groupe aryle contenant de 6 à 10 atomes de carbone, qui est éventuellement substitué une ou plusieurs fois par un atome d'halogène, par un groupe nitro, par un groupe cyano ou encore par un groupe alkyle ou par un groupe alcoxy à chaîne droite ou ramifiée contenant jusqu'à 8 atomes de carbone,

$R^1$ et $R^2$ forment ensemble un radical carbocyclique saturé ou insaturé contenant de 4 à 8 chaînons qui est éventuellement substitué jusqu'à 3 fois de manière identique ou différente par un groupe alkyle ou par un groupe alcoxy à chaîne droite ou ramifiée contenant chacun jusqu'à 8 atomes de carbone, par un atome d'halogène, par un groupe hydroxyle ou par un groupe aryle contenant de 6 à 10 atomes de carbone, ou

$R^1$ et $R^2$ représentent, conjointement avec l'atome de carbone, un radical de formule

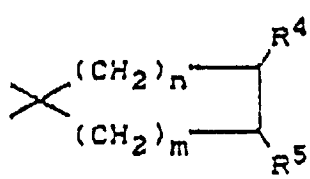

dans laquelle

m et n sont identiques ou différents et désignent le nombre 0, 1, 2, 3 ou 4,

et

$R^4$ et $R^5$ forment ensemble un groupe aryle contenant de 6 à 10 atomes de carbone ou un radical hétérocyclique penta- à heptagonal, saturé ou insaturé, contenant jusqu'à 3 hétéroatomes choisis parmi la série comprenant un atome d'azote, un atome de soufre ou un atome d'oxygène, qui sont éventuellement substitués une ou plusieurs fois par un groupe alkyle ou par un groupe alcoxy à chaîne droite ou ramifiée contenant chacun jusqu'à 8 atomes de carbone, par un atome d'halogène ou par un groupe aryle contenant de 6 à 10 atomes de carbone,

$R^3$ représente un atome d'hydrogène, un groupe alkyle à chaîne droite ou ramifiée contenant jusqu'à 8 atomes de carbone ou encore un groupe phényle,

et de leur sels, caractérisé en ce qu'on fait réagir des composés CH-acides répondant à la formule générale

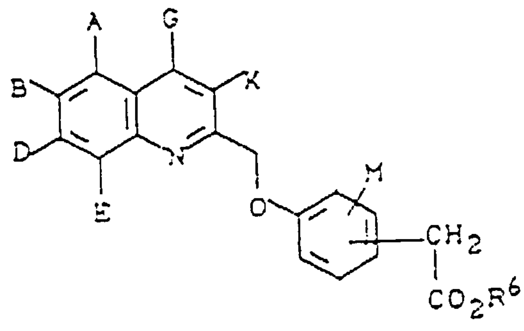

(II)

dans laquelle

A, B, D, E, G, K et M ont la signification indiquée ci-dessus,

et

$R^6$ a la signification de $R^3$ indiquée ci-dessus, mais ne représente pas un atome d'hydrogène,

avec des composés dihalogénés répondant à la formule générale (III)

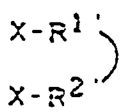

(III)

dans laquelle

$R^{1'}$ et $R^{2'}$ ont la signification indiquée ci-dessus pour $R^1$ et $R^2$, mais sont présents sous la forme d'une chaîne ouverte,

et

X représente un atome d'halogène,

dans des solvants inertes, éventuellement en présence d'une base, et ensuite, on saponifie les esters dans le cas des acides.